# EUROPEAN PATENT APPLICATION

(11) **EP 4 537 781 A1**
(43) Date of publication of application: **16.04.2025**
(21) Application number: 23203380.3
(22) Date of filing: 13.10.2023
(51) Int. Cl.: A61B 34/20, A61B 17/00, A61B 90/00

(54) **INTERVENTIONAL DEVICE POSITIONING**

(71) Applicant: Koninklijke Philips N.V., 5656 AG Eindhoven (NL)
(72) Inventor: SCHADEWALDT, Nicole, Eindhoven (NL); SCHULZ, Heinrich, Eindhoven (NL); NORDHOFF, Tanja, 5656AG Eindhoven (NL); JOCKEL, Sascha Andreas, Eindhoven (NL); WIEBERNEIT, Nataly, Eindhoven (NL); SAALBACH, Axel, Eindhoven (NL)
(74) Representative: Philips Intellectual Property & Standards

(57) **Abstract**

A system for assisting the positioning of an interventional device (110) in an anatomical region, is provided. The system includes one or more processors configured to analyse one or more X-ray projection images (130) to provide a comparison between a relative position of at least a portion (110') of the interventional device (110) with respect to one or more anatomical features (140) of the anatomical region, and a target relative position of the at least a portion (110') of the interventional device (110) with respect to the one or more anatomical features (140) of the anatomical region. Feedback is outputted for assisting the positioning of the interventional device (110) in the anatomical region. The feedback is generated based on the comparison.

## Description

### FIELD OF THE INVENTION

The present disclosure relates to assisting the positioning of an interventional device in an anatomical region. A system, an X-ray projection imaging system, a computer-implemented method, and a computer program product, are disclosed.

### BACKGROUND OF THE INVENTION

Various medical procedures involve the positioning of interventional devices in the anatomy. For instance, endotracheal tubes "ETTs", and tracheostomy tubes are often positioned in an airway to supply the lungs with oxygen. Drainage tubes, also known as chest tubes, are often positioned in the pleural space, or in the lungs, in order to drain air or fluid. Central venous catheters "CVCs" are often positioned in blood vessels in order to administer medicine to the bloodstream. Nasogastric tubes "NGTs", and other so-called "feeding tubes" are often positioned in the digestive tract in order to administer nutrients to the digestive system. Likewise, interventional devices such as nasopharyngeal airway tubes "NPATs", intercostal tube/catheters "ICCs", permanent pacemakers "PPMs", temperature probes, and pulmonary artery (e.g. Swan-Ganz) catheters, are often positioned in the anatomy during medical procedures.

After a physician has positioned such interventional devices in the anatomy, their positioning is typically verified using X-ray projection images. The images are typically reviewed by a radiologist who is trained to read such images. However, time pressure and clinical workflow boundary conditions can lead to a delay before the radiologist is able to review the images. Consequently, the physician that positions the interventional device may also verify its positioning by reviewing the images themself. This presents challenges because the physician may lack the formal training that is required to read the images. This challenge is often exacerbated by the poor quality of the images that are typical for urgent clinical settings like ICU with multi-morbid patients. Interventional devices such as those mentioned above are typically used in subjects with serious illnesses, and who consequently have limited mobility. The subject may therefore be unable to position themselves in a manner that is optimal for obtaining high quality images. Additional medical devices, so-called lines and tubes may also hinder optimal image acquisition. On the other hand, the clinical workflow with a seriously ill patient in a setting requiring urgent actions may require fast feedback on the correctness of the positioned device in spite of the poor quality of the images.

Consequently, there is a need to assist physicians in the assessment of interventional device positions in the anatomy.

### SUMMARY OF THE INVENTION

According to one aspect of the present disclosure, a system for assisting the position assessment of an interventional device in an anatomical region, is provided. The system comprises one or more processors configured to:
- receive X-ray image data comprising one or more X-ray projection images representing the interventional device in the anatomical region;
- analyse the one or more X-ray projection images to provide a comparison between a relative position of at least a portion of the interventional device with respect to one or more anatomical features of the anatomical region, and a target relative position of the at least a portion of the interventional device with respect to the one or more anatomical features of the anatomical region; and
- output feedback for assisting the positioning of the interventional device in the anatomical region, wherein the feedback is generated based on the comparison.

In the above system, the outputted feedback is determined based on a comparison between a relative position of a portion of the interventional device with respect to one or more anatomical features of the anatomical region, and a target relative position of the portion of the interventional device with respect to the one or more anatomical features of the anatomical region. This comparison provides a reliable assessment of the suitability of the position of the portion of the interventional device, and consequently the system provides reliable feedback. A physician may therefore use the feedback to determine a subsequent course of action with regard to the position of the interventional device without necessarily requiring the images to be reviewed by a radiologist.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for assisting the positioning of an interventional device in an anatomical region, in accordance with some aspects of the present disclosure.
Fig. 2 is a flowchart illustrating an example of a computer-implemented method of assisting the positioning of an interventional device in an anatomical region, in accordance with some aspects of the present disclosure.
Fig. 3 is an example of an X-ray projection image 130 representing an interventional device 110 in an anatomical region, in accordance with some aspects of the present disclosure.
Fig. 4 illustrates an X-ray projection image 130 including some examples of feedback 150 for assisting the positioning of an interventional device 110 in an anatomical region, in accordance with some aspects of the present disclosure.
Fig. 5 illustrates an X-ray projection image 130 including some further examples of feedback 150 for assisting the positioning of an interventional device 110 in an anatomical region, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in computer program product, in a corresponding manner.

In the following description, reference is made to a system for assisting the positioning of an interventional device in an anatomical region. In some examples, the interventional device is an endotracheal tube, i.e. an ETT. The ETT is positioned in an airway in order to supply the lungs with oxygen. However, it is to be appreciated that the system may also be used to assist the positioning of other types of interventional devices than an ETT, and that these may be positioned in other anatomical regions than an airway. For instance, the system may also be used to assist the positioning of tracheostomy tubes in an airway, to assist the positioning of drainage tubes, also known as chest tubes, in the pleural space, or in the lungs, to assist the positioning of central venous catheters "CVCs" in blood vessels, and to assist the positioning of nasogastric tubes "NGTs", and other so-called "feeding tubes" in the digestive tract. Likewise, the system may be used to assist the positioning of interventional devices in the anatomy such as nasopharyngeal airway tubes "NPATs", intercostal tube/catheters "ICCs", permanent pacemakers "PPMs", temperature probes, pulmonary artery (e.g. Swan-Ganz) catheters, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact diskread only memory "CD-ROM", compact disk-read/write "CD-R/W", Blu-Ray^{™} and DVD.

It is also noted that some operations that are performed by the one or more processors of the system disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there is a need to assist physicians in the positioning of interventional devices in the anatomy.

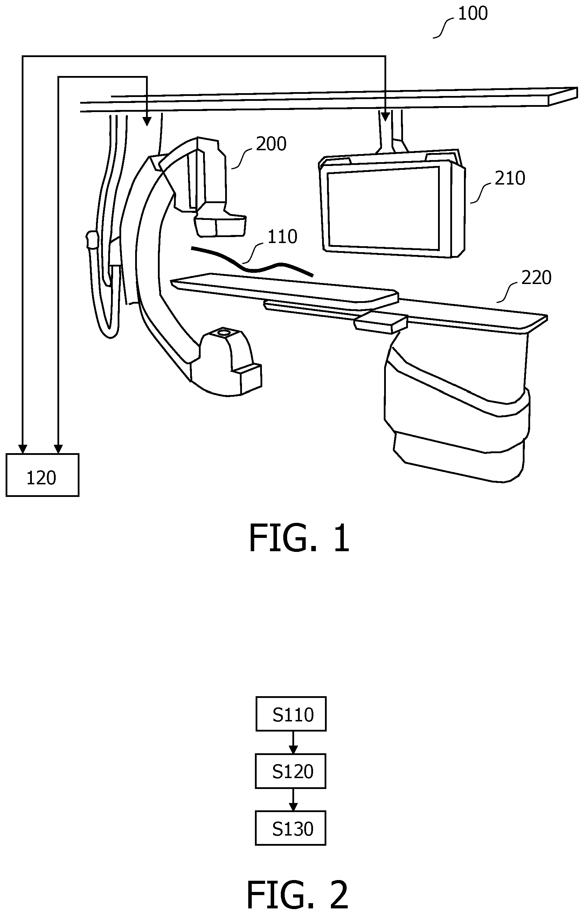

Fig. 1 is a schematic diagram illustrating an example of a system 100 for assisting the positioning of an interventional device in an anatomical region, in accordance with some aspects of the present disclosure. Fig. 2 is a flowchart illustrating an example of a computer-implemented method of assisting the positioning of an interventional device in an anatomical region, in accordance with some aspects of the present disclosure. It is noted that operations described as being performed by the one or more processors 120 of the system 100 illustrated in Fig. 1, may also be performed in the method illustrated in Fig. 2. Likewise, operations described in relation to the method described with reference to Fig. 2 may also be performed by the one or more processors 120 of the system 100 illustrated in Fig. 1.

With reference to Fig. 1, and Fig. 2, the system 100 for assisting the positioning of an interventional device 110 in an anatomical region, comprises one or more processors 120 configured to:
- receive S110 X-ray image data comprising one or more X-ray projection images 130 representing the interventional device 110 in the anatomical region;
- analyse S120 the one or more X-ray projection images 130 to provide a comparison between a relative position of at least a portion 110' of the interventional device 110 with respect to one or more anatomical features 140 of the anatomical region, and a target relative position of the at least a portion 110' of the interventional device 110 with respect to the one or more anatomical features 140 of the anatomical region; and
- output S130 feedback 150 for assisting the positioning of the interventional device 110 in the anatomical region, wherein the feedback 150 is generated based on the comparison.

In the above system, the outputted feedback is determined based on a comparison between a relative position of a portion of the interventional device with respect to one or more anatomical features of the anatomical region, and a target relative position of the portion of the interventional device with respect to the one or more anatomical features of the anatomical region. This comparison provides a reliable assessment of the suitability of the position of the portion of the interventional device, and consequently the system provides reliable feedback. A physician may therefore use the feedback to determine a subsequent course of action with regard to the position of the interventional device without necessarily requiring the images to be reviewed by a radiologist.

The operations that are performed by the one or more processors of the system 100 are described in more detail below.

In the operation S 110, X-ray image data is received. The X-ray image data comprises one or more X-ray projection images 130 representing an interventional device 110 in an anatomical region.

The X-ray image data that is received in the operation S110 may be generated by various types of X-ray projection imaging systems. These include conventional X-ray projection imaging systems which generate X-ray attenuation data representing X-ray attenuation within a single energy interval, and spectral X-ray projection imaging systems which generate X-ray attenuation data representing X-ray attenuation within multiple different energy intervals. The X-ray attenuation data generated by spectral X-ray projection imaging systems may be processed, e.g. using various material decomposition algorithms, in order to distinguish between media that have similar X-ray attenuation values when measured within a single energy interval, and which would be indistinguishable in X-ray attenuation data obtained using conventional X-ray projection imaging systems. The X-ray attenuation data generated by spectral X-ray projection imaging systems may therefore be used to provide images with improved specificity to materials such as tissue, bone, and so forth.

The X-ray image data that is received in the operation S110 may be generated by various configurations of X-ray projection imaging systems. These include X-ray projection imaging systems that include a support arm such as a so-called "C-arm" or a so-called "O-arm" that supports an X-ray source and an X-ray detector, and also X-ray projection imaging systems in which the X-ray source and the X-ray detector are supported in a different manner. An example of an X-ray projection imaging system that includes a C-arm is the Azurion 7 X-ray X-ray projection imaging system that is marketed by Philips Healthcare, Best, The Netherlands. An example of an X-ray projection imaging system in which the X-ray source and the X-ray detector are supported in a different manner is the DigitalDiagnost C90 imaging system that is marketed by Philips Healthcare, Best, The Netherlands.

With continued reference to the operation S110, the X-ray image data that is received in the operation S110 comprises one or more X-ray projection images 130 representing an interventional device 110 in the anatomical region. In some examples, a single X-ray projection image 130, is received. In other examples, multiple X-ray images are received. For instance, a temporal sequence of images may be received. The temporal sequence of images may a live temporal sequence of images, or a pre-recorded temporal sequence of images.

As mentioned above, various types of intervention devices may be represented in the one or more X-ray projection images 130 that are received in the operation S110. In some examples, the interventional device is an interventional therapeutic device. In some examples, the interventional device 110 comprises a tube configured for insertion into an airway or a digestive tract or a pleural space of a subject, or a catheter configured for insertion into a blood vessel of a subject. By way of some examples, the interventional device may be an endotracheal tube, i.e. an ETT. The ETT may be positioned in an airway in order to supply the lungs with oxygen. By way of some further examples, the interventional device may alternatively be a tracheostomy tube. The tracheostomy tube may be positioned in an airway. The interventional device may alternatively be a drainage tubes, also known as chest tube. The drainage tube, or chest tube, may be positioned in the pleural space, or in a lung. The interventional device may alternatively be a central venous catheters "CVC". The CVC may be positioned in a blood vessel. The interventional device may alternatively be a nasogastric tube "NGT", also known as a "feeding tube". The NGT, or the feeding tube, may be positioned in the digestive tract. The interventional device may alternatively be a device such as a nasopharyngeal airway tube "NPAT", an intercostal tube/catheter "ICC", a permanent pacemaker "PPM", a temperature probe, a pulmonary artery (e.g. Swan-Ganz) catheter, and so forth.

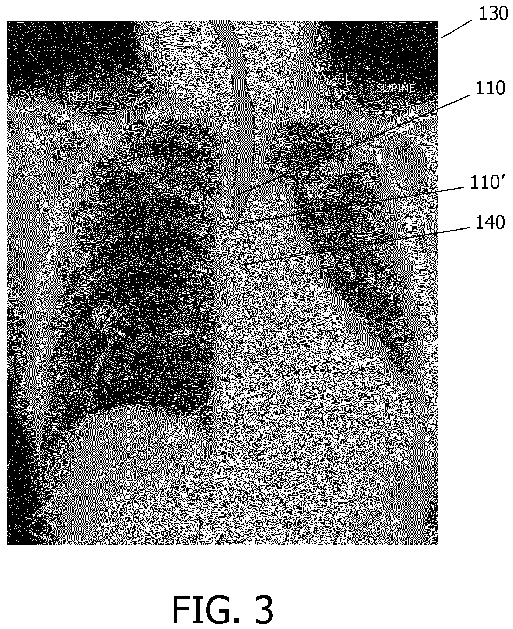

Fig. 3 is an example of an X-ray projection image 130 representing an interventional device 110 in an anatomical region, in accordance with some aspects of the present disclosure. In the example illustrated in Fig. 3, a single X-ray projection image 130 represents an ETT 110 in the thoracic region of a subject. Thus, in this example, the interventional device is an ETT, and the anatomical region is the thoracic region. More specifically, the ETT 110, i.e. an example of a therapeutic device, is located in an airway of the subject, and is used to supply the lungs with oxygen.

With continued reference to the operation S110, the X-ray image data that is received in the operation S110 may be received from various sources. For example, the X-ray image data, may be received from an X-ray projection imaging system, such as one of the example X-ray projection imaging systems described above, or it may be received from another source, such as from a computer-readable storage medium, or from the internet, or from the cloud, and so forth. The X-ray image data may be received via any form of data communication, including via wired or wireless communication. By way of some examples, when wired communication is used, the communication may take place via an electrical or optical cable, and when wireless communication is used, the communication may for example take place via RF or infrared signals.

Referring now to the operation S120 mentioned above with reference to Fig. 1 and Fig. 2, in this operation, the one or more X-ray projection images 130 are analysed to provide a comparison between a relative position of at least a portion 110' of the interventional device 110 with respect to one or more anatomical features 140 of the anatomical region, and a target relative position of the at least a portion 110' of the interventional device 110 with respect to the one or more anatomical features 140 of the anatomical region.

In general, the portion 110' of the interventional device 110 that is used in the comparison in the operation S120 may be any portion of the interventional device. The portion may be the tip, also known as a distal end, of the interventional device, or another portion of the interventional device such as a radiopaque marker disposed on the interventional device, for example. By way of some examples, in the example of the interventional device being an ETT, the portion may be the tip of the ETT. In the example of the interventional device being an NGT, the portion may likewise be the tip of the NGT since it is important to position the tip in the upper part of the stomach. The portion of the NGT may also include a portion of the body of the NGT since it is important to ensure that the body of the NGT is in the esophagus. In the example of the interventional device being a CVC, the portion may likewise be the tip of the CVC since it is important to ensure that the tip is in the correct position, and not, for example, directed towards a vessel wall. The portion of the CVC may also include a portion of the body of the CVC since it is useful to verify that the CVC is in the correct vessel, and not, for example, simply appearing to be projected onto the correct position in the X-ray projection images.

In general, the one or more anatomical features 140 that are used in the comparison in the operation S120 may be any anatomical features. The anatomical features may for example be a portion of a bone, e.g. a clavicle, or a rib, or a vertebra. In some examples, the anatomical region may include a tubular structure, or a cavity, or an anatomical surface, and the one or more anatomical features 140 may include at least one anatomical feature of the tubular structure, or the cavity, or the anatomical surface, respectively. By way of some examples, the tubular structure may for instance be an airway, or a portion thereof such as the trachea, or the bronchi, or a portion of the digestive tract, or a blood vessel. The cavity may for example be the lung, or the stomach, or a cardiac cavity such as the atrium. The anatomical surface may for example be the diaphragm, or a surface of an airway, or a surface of the digestive tract, or a surface of a blood vessel, or a surface of the lung, or a surface of the stomach, or a surface of a cardiac cavity. The anatomical feature of the tubular structure, or the cavity, or the anatomical surface, may be a prominence, a ridge, a bifurcation, or a start, or an end, of the tubular structure, or the cavity, or the anatomical surface, for example.

With reference to the example illustrated in Fig. 3, in this example, the operation S120 may include analysing the X-ray projection image 130 to provide a comparison between a relative position of a distal end 110' of the ETT 110 with respect to the tracheal carina 140, and a target relative position of a distal end 110' of the ETT 110 with respect to the tracheal carina 140. The tracheal carina 140 is a ridge of cartilage at the base of the trachea separating the openings of the left and right main bronchi, and may serve as the anatomical feature for use in positioning the distal end 110' of the ETT 110 in the airway. In this example, the relative position may be determined in the form of a measurement of a separation between the distal end 110' of the ETT 110 and the tracheal carina 140. The separation may be measured in units such as centimetres, or pixels, or another unit. Alternatively, or additionally, other anatomical features may serve as anatomical features for use in the operation S120, including for example a distal end of the esophagus.

In the case of positioning other types of interventional devices, other anatomical features may serve as anatomical features for use in the operation S120. For instance, for correct positioning of an NGT, a suitable anatomic feature include part of the stomach, and the relative position may be determined in the form of a measurement of a separation between the distal end of the NGT and the stomach. For correct positioning of a CVC the anatomical feature may include part of the heart, and the relative position may be determined in the form of a measurement of a separation between the distal end of the CVC and the part of the heart in which the CVC should be located. Additionally, the relative position may be determined in the form of a desired angle between the CVC and the cardiac wall, or the vessel wall. The CVC should ideally be oriented approximately parallel to the vessel wall in order to avoid washing the medication that is delivered by the CVC directly into the wall.

Various approaches may be used to perform the operation S120. In general, the comparison may be evaluated using an algorithm. The algorithm may be evaluated based on various factors. In some cases a single factor may be used to evaluate the comparison. For example, the comparison may be evaluated based on a comparison between a measurement of a separation between the at least a portion 110' of the interventional device 110 and a single anatomical feature 140, and a target measurement of a separation between the at least a portion 110' of the interventional device 110 and a single anatomical feature 140. For instance, with reference to the example illustrated in Fig. 3, a measurement of a separation between the distal end 110' of the ETT 110 and the tracheal carina 140 may be evaluated in units of centimetres, or image pixels, and compared with a target value of the separation between the distal end 110' of the ETT 110 and the tracheal carina 140. In other cases, multiple factors may be used to evaluate the comparison. For example, the comparison may be evaluated based on a comparison between a measurement of a separation between the at least a portion 110' of the interventional device 110 and each of multiple anatomical features, and target measurements of the separation between the at least a portion 110' of the interventional device 110 and each of the anatomical features. The distal end of the esophagus may serve as an additional anatomical feature, for instance. When multiple factors are used to evaluate the comparison, the same, or different, weightings may be applied to the individual factors in order to reflect the relative importance of the factors.

In another example, the comparison that is performed in the operation S120 is evaluated based on a pattern rather than measurements.

For example, the comparison may be evaluated by comparing a pattern formed by the positions of the at least a portion 110' of the interventional device 110 and the one or more anatomical features, with a target pattern formed by the positions of the at least a portion 110' of the interventional device 110 and the one or more anatomical features. In this example, the comparison may be made using various image similarity metrics that are known from the image processing field. The comparison of such patterns may be seen as the comparison of constellations of positions.

In another example, the comparison that is performed in the operation S120 is evaluated using a neural network. In this example, the one or more processors 120 are configured to analyse S120 the one or more X-ray projection images 130 by:
- inputting the X-ray image data into a neural network; and
- generating the feedback 150, using the neural network, in response to the inputting; and
- wherein the neural network is trained to generate the feedback 150 using training data comprising a plurality of X-ray projection training images, and for each X-ray projection training image, corresponding ground truth feedback for assisting the positioning of the interventional device 110 in the anatomical region, the ground truth feedback for each X-ray projection training image being based on a comparison between a relative position of at least a portion 110' of the interventional device 110 in the training image with respect to one or more anatomical features of the anatomical region in the training image, and a target relative position of the at least a portion 110' of the interventional device 110 with respect to the one or more anatomical features of the anatomical region.

Various types of feedback that may be provided by the neural network are described below. The corresponding ground truth feedback that is used to train the neural network may be provided by a medical professional such as a radiologist. For instance, the radiologist may annotate the X-ray projection training images with the positions of a distal end of the interventional device, and one or more anatomical features. The medical professional may further annotate the X-ray projection training images with feedback. The feedback may include qualitative feedback such as "relative position is/ is not acceptable" and/or quantitative feedback regarding the relative distance between the distal end the interventional device and the anatomical features, such as "The ETT is correctly positioned 2.5 centimetres above the carina", and so forth.

The neural network in this example may be provided by various architectures, including a convolutional neural network, "CNN", a recurrent neural network "RNN", or a transformer, and so forth. In general, the training of the neural network involves inputting the training data into the neural network, and iteratively adjusting the neural network's parameters until the trained neural network provides an accurate output. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative loglikelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

The training of a neural network is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

In the above example, the neural network may be trained to generate the feedback 150 by:
- for each of a plurality of the X-ray projection training images:
- inputting the X-ray projection training image into the neural network;
- generating feedback 150 for assisting the positioning of the interventional device 110 in the anatomical region, using the neural network, in response to the inputting; and
- adjusting parameters of the neural network based on a difference between the feedback generated by the neural network, and the corresponding ground truth feedback for the X-ray projection training image; and
- repeating the inputting, the generating, and the adjusting, until a stopping criterion is met.

It is noted that in the above examples, the operation S120 may be preceded by a further operation of identifying the at least a portion 110' of the interventional device 110, and the one or more anatomical features 140. This operation may be performed using segmentation algorithms such as modelbased segmentation, watershed-based segmentation, region growing or level sets or graphcuts. A machine learning technique may also be used to identify the at least a portion 110' of the interventional device 110. An example of a machine learning technique that may be used for this purpose is disclosed in a document by Jiang, P., et al., "A review of Yolo algorithm developments", Procedia Computer Science, Vol. 199, 2022, pages 1066 - 1073.

The one or more processors 120 may also receive input defining a threshold value for the comparison. The feedback 150 may then be generated based on the threshold value for the comparison. This facilitates the setting of the threshold for how closely the relative position of at least a portion 110' of the interventional device 110 with respect to one or more anatomical features 140 of the anatomical region, should conform to the target relative position of the at least a portion 110' of the interventional device 110, in order to obtain a desired level of feedback. This example facilitates a clinical facility to set the threshold value according to its protocols.

The one or more processors may also generate a confidence measure for the comparison. The value of the confidence measure may be outputted by the system. The confidence score helps to reassure a physician that the feedback that is provided by the system is reliable. The confidence measure may be evaluated using various techniques. The confidence measure may for example be determined based on factors such as an image quality measure across the one or more X-ray projection images, an image quality measure in the region of interest in the one or more X-ray projection images, a specific image feature score such as the average magnitude of a gradient at the boundary of a structure of interest in the one or more X-ray projection images, a probability score (local or integral) outputted by a neural network that detected the device, a probability score for other detected interventional devices, anatomical target structures, anatomical neighbouring structures, or detected pathologies that might obscure the device, or background information on the visibility of the interventional device.

A combined confidence measure might be computed from the above-mentioned factors. For example, in one implementation, the average probability score of the interventional device localization outputted by a neural network. In another implementation, the above mentioned probability scores may be used to lower the combined confidence measure if they are below specified threshold values, e.g. the combined confidence measure may be calculated as the minimum of the other confidences. The combined confidence measure may be computed based on the probability scores such as those mentioned above using a neural network that is trained on radiologist-scored confidence values.

Referring now to the operation S130 mentioned above with reference to Fig. 1 and Fig. 2, in this operation, feedback 150 is outputted for assisting the positioning of the interventional device 110 in the anatomical region. The feedback 150 is generated based on the comparison.

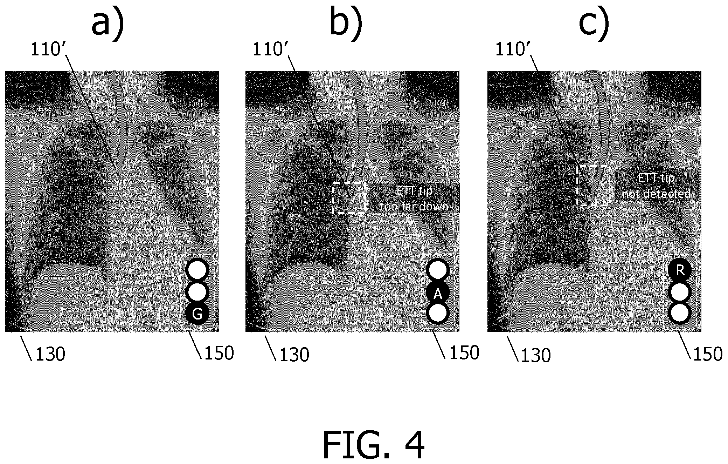

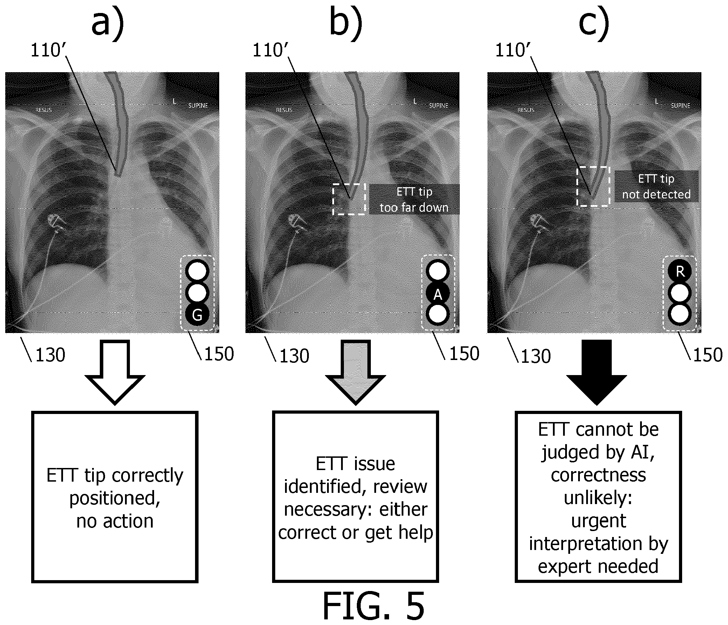

Various types of feedback 150 may be outputted in the operation S130. These include the outputting of "traffic light" feedback, and the outputting of messages. Some examples of these types of feedback are described below with reference to Fig. 4 and Fig. 5. Fig. 4 illustrates an X-ray projection image 130 including some examples of feedback 150 for assisting the positioning of an interventional device 110 in an anatomical region, in accordance with some aspects of the present disclosure. Fig. 5 illustrates an X-ray projection image 130 including some further examples of feedback 150 for assisting the positioning of an interventional device 110 in an anatomical region, in accordance with some aspects of the present disclosure.

In one example, the feedback 150 comprises an indication of a compliance and/or a deviation between the relative position of the at least a portion 110' of the interventional device 110 with respect to one or more anatomical features of the anatomical region in the one or more X-ray projection images 130, and the target relative position of the at least a portion 110' of the interventional device 110 with respect to the one or more anatomical features of the anatomical region.

In this example, the feedback may simply be provided in the form of a binary "yes/ no" indication. For example, a "yes" indication may be provided if the relative position of the at least a portion 110' of the interventional device 110 with respect to one or more anatomical features of the anatomical region in the one or more X-ray projection images 130, is within a specified margin of the target relative position. An example of this type of feedback is illustrated in Fig. 4a), and wherein a green "traffic light" indication "G" is provided. This indication may be provided if, for example, the measurement of the separation between the distal end 110' of the ETT 110 and the tracheal carina 140, is within a specified margin. Alternatively, a "no" indication may be provided if the relative position of the at least a portion 110' of the interventional device 110 with respect to one or more anatomical features of the anatomical region in the one or more X-ray projection images 130, deviates from the target relative position by more than a specified margin. In this case, instead of providing a green traffic light indication as illustrated in Fig. 4a), an amber traffic light indication "A" may be provided, as illustrated in Fig. 4b). Providing the feedback in this straightforward manner allows the physician to determine the course of action without necessarily requiring the image to be reviewed by a radiologist. For instance, the physician may be triggered by the amber traffic light indication to re-position the interventional device in an improved manner themselves.

In this example, instead of, or in addition to providing a binary "yes/ no" indication in the form of the above-described traffic light indication, a binary "yes/ no" indication may be provided in the form of a message. An example of this type of feedback is illustrated in Fig. 5a), and wherein the message indicates that the "ETT tip is correctly positioned", and also in Fig. 5b) and wherein the message "ETT issue identified, review necessary".

In this example, instead of providing the feedback in the form of a binary "yes/ no" indication, further granularity may be provided. For example, the feedback 150 may include an indication of a magnitude of compliance and/or a magnitude of a deviation between the relative position of the at least a portion 110' of the interventional device 110 with respect to one or more anatomical features of the anatomical region in the one or more X-ray projection images 130, and the target relative position of the at least a portion 110' of the interventional device 110 with respect to the one or more anatomical features of the anatomical region. In this example, the feedback may for example include a message representing a magnitude of the magnitude of compliance and/or a magnitude of a deviation. With reference to the example illustrated in Fig. 4b), and in Fig. 5b), the feedback may be provided in qualitative terms, for instance in the form of a message indicating that the "ETT tip is too far down" in the airway. Alternatively, the feedback may indicate the magnitude of the deviation in quantitative terms, such as "ETT tip is 5 centimetres above target position".

In another example, the feedback comprises an alert for alerting a radiologist of the need to review the positioning of the interventional device 110 in the one or more X-ray projection images 130. Feedback may be provided in the form of the alert in situations in which there is uncertainty in the comparison. For instance, it may be provided if the confidence measure for the comparison is below a threshold value. The alert may be generated in situations in which it is difficult to accurately detect the portion 110' of the interventional device 110 and/or the anatomical feature(s) with respect to which its position is assessed. For instance, the X-ray projection image may have been generated from a suboptimal imaging direction that results in the obstruction of these image features, or there might be overlays of other internal or external devices on the device of interest which prohibits a suitable evaluation. By way of some examples, the alert may be provided in situations such as the portion of the interventional device, e.g. the distal end 110' of the ETT 110, not being detected accurately, and/or the anatomical features 140 of the anatomical region, e.g. the tracheal carina 140, not being detected accurately.

Providing the alert facilitates an assessment of the positioning of the interventional device to be deferred to the radiologist, and avoids the risk that the system provides a recommendation that is based on comparison that is potentially inaccurate.

An example of such an alert is illustrated in Fig. 4c) and wherein the red "traffic light" indication "R" is provided. Another example of such an alert is illustrated in Fig. 5c) and wherein an alert is illustrated via the message "urgent interpretation by expert needed".

In a related example, the alert may also include a priority level, such as high/ low priority, for example. The priority level facilitates a radiologist to prioritize the reading the image, and thereby efficiently enable the physician to make an appropriate medical intervention, should this be necessary.

In another example, the feedback 150 comprises a recommendation for obtaining an improved position of the at least a portion 110' of the interventional device 110 with respect to one or more anatomical features. An example of this type of feedback is to provide an instruction to "advance/ retract the ETT by 2 centimetres" so as to position the tip of the ETT within a specified distance of the target position. Providing the feedback in this manner facilitates a physician to rectify a mal-positioned interventional device.

In a related example, if the anatomical region comprises a tubular structure, or a cavity, or an anatomical surface; the feedback 150 may include an instruction to advance or retract the interventional device 110 within the tubular structure, or within the cavity, or with respect to the anatomical surface, respectively. The feedback may include an instruction to advance or retract the interventional device 110 by a specified amount. For instance, with reference to the example illustrated in Fig. 4b), the feedback may include an instruction to "retract the ETT by 2 centimetres" so as to position the tip of the ETT within a specified distance of the target position.

In another example, the feedback 150 comprises i) an indication of an inability to accurately compare the relative position of at least a portion 110' of the interventional device 110 with the target relative position of the at least a portion 110' of the interventional device 110, or ii) a recommendation to obtain one or more improved X-ray projection images 130 representing the interventional device 110 in the anatomical region.

An example of the former type of feedback is illustrated in Fig. 4c) via the message "ETT tip not detected". The feedback that is provided by this example facilitates the physician to determine the subsequent course of action without risking a reliance on an inaccurate assessment of the positioning of the interventional device. In this example, a bounding box may be provided in the X-ray projection image 130 to indicate the region in which the comparison that is made in the operation S120 is inaccurate, as also illustrated in Fig. 4c).

In another example, the one or more processors 120 are configured to evaluate a confidence measure for the comparison, and the feedback 150 is generated based further on the value of the confidence measure. For instance if the value of the confidence measure is below a threshold value, feedback may be provided in the form of the alert described above. The value of the confidence measure may be calculated using the techniques described above.

The feedback 150 that is outputted in the operation S130 may be outputted in various ways, including visually, and audially. Visual feedback may be provided via a display such as via the display 210 illustrated in Fig. 1, or via one or more indicator lamps, for example. In one example, the one or more processors 120 are configured to output the one or more X-ray projection images 130, and the feedback 150 is displayed in the outputted one or more X-ray projection images 130. The feedback may be provided in the form or a message, or an icon, such as the messages, and the traffic light icon, described above. The feedback may be displayed in the X-ray projection image(s) in the form of an overlay, for example. Examples of this type of feedback are illustrated in Fig. 4a) - Fig. 4c) above.

It is noted that in the examples described above, the system 100 may also include one or more of: an X-ray projection imaging system for providing the X-ray image data, such as for example the X-ray projection imaging system 200 illustrated in Fig. 1; an interventional device, such as the ETT 110 illustrated in Fig. 1; a monitor 210 for displaying the feedback 150, the one or more X-ray projection images 130, and other outputs generated by the one or more processors 120; a patient bed 220; and a user input device (not illustrated in Fig. 1) configured to receive user input in relation to the operations performed by the one or more processors 120, such as a keyboard, a mouse, a touchscreen, and so forth.

In another example, a computer-implemented method of assisting the positioning of an interventional device 110 in an anatomical region, is provided. The method comprises:
- receiving S110 X-ray image data comprising one or more X-ray projection images 130 representing the interventional device 110 in the anatomical region;
- analysing S120 the one or mores X-ray projection images 130 to provide a comparison between a relative position of at least a portion 110' of the interventional device 110 with respect to one or more anatomical features 140 of the anatomical region, and a target relative position of the at least a portion 110' of the interventional device 110 with respect to the one or more anatomical features 140 of the anatomical region; and
- outputting S130 feedback 150 for assisting the positioning of the interventional device 110 in the anatomical region, wherein the feedback 150 is generated based on the comparison.

In another example, a computer program product is provided. The computer program product comprises instructions which when executed by one or more processors 120, cause the one or more processors to carry out a method of assisting the positioning of an interventional device 110 in an anatomical region. The method comprises:
- receiving S110 X-ray image data comprising one or more X-ray projection images 130 representing the interventional device 110 in the anatomical region;
- analysing S120 the one or mores X-ray projection images 130 to provide a comparison between a relative position of at least a portion 110' of the interventional device 110 with respect to one or more anatomical features 140 of the anatomical region, and a target relative position of the at least a portion 110' of the interventional device 110 with respect to the one or more anatomical features 140 of the anatomical region; and
- outputting S130 feedback 150 for assisting the positioning of the interventional device 110 in the anatomical region, wherein the feedback 150 is generated based on the comparison.

In another example, an X-ray projection imaging system 200, is provided. The X-ray projection imaging system 200 comprises one or more processors 120 configured to:
- receive S110, from the X-ray projection imaging system 200, X-ray image data comprising one or more X-ray projection images 130 representing an interventional device 110 in an anatomical region;
- analyse S120 the one or more X-ray projection images 130 to provide a comparison between a relative position of at least a portion 110' of the interventional device 110 with respect to one or more anatomical features 140 of the anatomical region, and a target relative position of the at least a portion 110' of the interventional device 110 with respect to the one or more anatomical features 140 of the anatomical region; and
- output S130 feedback 150 for assisting the positioning of the interventional device 110 in the anatomical region, wherein the feedback 150 is generated based on the comparison.

The above examples are to be understood as illustrative of the present disclosure, and not restrictive. Further examples are also contemplated. For instance, the examples described in relation to a system 100, may also be provided by the X-ray projection imaging system 200, or by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for assisting the positioning of an interventional device (110) in an anatomical region, the system comprising one or more processors (120) configured to:
receive (S110) X-ray image data comprising one or more X-ray projection images (130) representing the interventional device (110) in the anatomical region;
analyse (S120) the one or more X-ray projection images (130) to provide a comparison between a relative position of at least a portion (110') of the interventional device (110) with respect to one or more anatomical features (140) of the anatomical region, and a target relative position of the at least a portion (110') of the interventional device (110) with respect to the one or more anatomical features (140) of the anatomical region; and
output (S130) feedback (150) for assisting the positioning of the interventional device (110) in the anatomical region, wherein the feedback (150) is generated based on the comparison.

2. The system according to claim 1, wherein the anatomical region comprises a tubular structure, or a cavity, or an anatomical surface, and wherein the one or more anatomical features (140) comprise at least one anatomical feature of the tubular structure, or the cavity, or the anatomical surface, respectively.

3. The system according to any previous claim, wherein the interventional device (110) comprises a tube configured for insertion into an airway or a digestive tract or a pleural space of a subject, or a catheter configured for insertion into a blood vessel of a subject.

4. The system according to any previous claim, wherein the one or more processors (120) are configured to analyse (S120) the one or more X-ray projection images (130) by:
inputting the X-ray image data into a neural network; and
generating the feedback (150), using the neural network, in response to the inputting; and
wherein the neural network is trained to generate the feedback (150) using training data comprising a plurality of X-ray projection training images, and for each X-ray projection training image, corresponding ground truth feedback for assisting the positioning of the interventional device (110) in the anatomical region, the ground truth feedback for each X-ray projection training image being based on a comparison between a relative position of at least a portion (110') of the interventional device (110) in the training image with respect to one or more anatomical features of the anatomical region in the training image, and a target relative position of the at least a portion (110') of the interventional device (110) with respect to the one or more anatomical features of the anatomical region.

5. The system according to claim 4, wherein the neural network is trained to generate the feedback (150) by:
for each of a plurality of the X-ray projection training images:
inputting the X-ray projection training image into the neural network;
generating feedback (150) for assisting the positioning of the interventional device (110) in the anatomical region, using the neural network, in response to the inputting; and
adjusting parameters of the neural network based on a difference between the feedback generated by the neural network, and the corresponding ground truth feedback for the X-ray projection training image; and
repeating the inputting, the generating, and the adjusting, until a stopping criterion is met.

6. The system according to any previous claim, wherein the one or more processors (120) are further configured to evaluate a confidence measure for the comparison; and
wherein the feedback (150) is generated based further on the value of the confidence measure.

7. The system according to any previous claim, wherein the one or more processors (120) are further configured to receive input defining a threshold value for the comparison; and
wherein the feedback (150) is generated based further on the threshold value for the comparison.

8. The system according to any previous claim, wherein the feedback (150) comprises an indication of a compliance and/or a deviation between the relative position of the at least a portion (110') of the interventional device (110) with respect to one or more anatomical features of the anatomical region in the one or more X-ray projection images (130), and the target relative position of the at least a portion (110') of the interventional device (110) with respect to the one or more anatomical features of the anatomical region.

9. The system according to any previous claim, wherein the feedback comprises an alert for alerting a radiologist of the need to review the positioning of the interventional device (110) in the one or more X-ray projection images (130).

10. The system according to any previous claim, wherein the feedback (150) comprises a recommendation for obtaining an improved position of the at least a portion (110') of the interventional device (110) with respect to one or more anatomical features.

11. The system according to claim 10, wherein the anatomical region comprises a tubular structure, or a cavity, or an anatomical surface; and
wherein the feedback (150) comprises an instruction to advance or retract the interventional device (110) within the tubular structure, or within the cavity, or with respect to the anatomical surface, respectively.

12. The system according to any previous claim, wherein the feedback (150) comprises i) an indication of an inability to accurately compare the relative position of at least a portion (110') of the interventional device (110) with the target relative position of the at least a portion (110') of the interventional device (110), or ii) a recommendation to obtain one or more improved X-ray projection images (130) representing the interventional device (110) in the anatomical region.

13. The system according to any previous claim, wherein the one or more processors (120) are further configured to output the one or more X-ray projection images (130); and
wherein the feedback (150) is displayed in the outputted one or more X-ray projection images (130).

14. A computer-implemented method of assisting the positioning of an interventional device (110) in an anatomical region, the method comprising:
receiving (S110) X-ray image data comprising one or more X-ray projection images (130) representing the interventional device (110) in the anatomical region;
analysing (S120) the one or mores X-ray projection images (130) to provide a comparison between a relative position of at least a portion (110') of the interventional device (110) with respect to one or more anatomical features (140) of the anatomical region, and a target relative position of the at least a portion (110') of the interventional device (110) with respect to the one or more anatomical features (140) of the anatomical region; and
outputting (S130) feedback (150) for assisting the positioning of the interventional device (110) in the anatomical region, wherein the feedback (150) is generated based on the comparison.

15. A computer program product comprising instructions which when executed by one or more processors (120), cause the one or more processors to carry out the method according to claim 14.

16. An X-ray projection imaging system (200) comprising one or more processors (120) configured to:
receive (S110), from the X-ray projection imaging system (200), X-ray image data comprising one or more X-ray projection images (130) representing an interventional device (110) in an anatomical region;
analyse (S120) the one or more X-ray projection images (130) to provide a comparison between a relative position of at least a portion (110') of the interventional device (110) with respect to one or more anatomical features (140) of the anatomical region, and a target relative position of the at least a portion (110') of the interventional device (110) with respect to the one or more anatomical features (140) of the anatomical region; and
output (S130) feedback (150) for assisting the positioning of the interventional device (110) in the anatomical region, wherein the feedback (150) is generated based on the comparison.
